# EUROPEAN PATENT APPLICATION

(11) **EP 1 770 385 A2**
(43) Date of publication of application: **04.04.2007**
(21) Application number: 06121482.1
(22) Date of filing: 29.09.2006
(51) Int. Cl.: G01N 3/00

(54) **Document durability testing apparatus**

(30) Priority: 30.09.2005 GB 0519970
(71) Applicant: De La Rue International Limited, Basingstoke, Hampshire RG22 4BS (GB)
(72) Inventor: Chamberlain, Adrian, Basingstoke Hampshire RG21 8YG (GB)
(74) Representative: Skone James, Robert Edmund

(57) **Abstract**

Document durability testing apparatus comprises:
a document pocket (31), in which a document and rubbing pad (41) are inserted in use, the document pocket being made of a flexible material and being coupled with attachment portions (34,35) at each end so that the pocket can be tensioned; and
a rubbing system (10,16) including a pushing member (16) which engages and moves in use relative to the document pocket (31), and which is mounted so as to urge portions of the pocket, document and rubbing pad adjacent the pushing member away from the pushing member thereby also causing relative rubbing movement between the document and the rubbing pad in the pocket.

## Description

The invention relates to a document durability testing apparatus, particularly for testing printed documents including documents of value such as banknotes.

When designing documents of value such as banknotes, it is important to be able to test their durability and in particular their ability to retain ink during normal use. A number of testing apparatus have been proposed in the past.

US-A-2734375 discloses one example which is embodied in a so-called "Sutherland" tester. In this apparatus, a rubber pad is mounted on a base and a banknote to be tested is placed on the pad where the rubber-like character of the pad retains the sample in place. A test block carrying a strip of test, abrasive paper is then brought to bear on the exposed surface of the banknote and moved to an fro over the surface. Although this apparatus works well with paper documents, we have found that it does not accurately simulate the normal wear problem found in circulation in respect of polymer documents such as plastic banknotes.

US-A-4462245 discloses a rub testing apparatus similar to that described above. In this case, however, a cylindrical rod is placed beneath the document so that the document is bent around the pin.

US-A-6477895 describes apparatus for determining the permanence of a toner image. In this case, a document bearing a toner image is located between a base and a receiver sheet, part of the receiver sheet being urged towards the document by a pressure pad. The document is then pulled out from between the receiver sheet and the base. This apparatus is not suitable for more general durability tests, particularly those concerned with the normal use of banknotes because it is specifically designed to simulate set-off or rubbing off of toner between two stacked sheets, for example to simulate problems where freshly copied sheets are fed into a stacker and then more sheets feed out on top into the stacker.

In accordance with the present invention, document durability testing apparatus comprises:
a document pocket, in which a document and rubbing pad are inserted in use, the document pocket being made of a flexible material and being coupled with attachment portions at each end so that the pocket can be tensioned; and
a rubbing system including a pushing member which engages and moves in use relative to the document pocket, and which is mounted so as to urge portions of the pocket, document and rubbing pad adjacent the pushing member away from the pushing member thereby also causing relative rubbing movement between the document and the rubbing pad in the pocket.

We have found that while the known rub testing apparatus works acceptably with paper-based documents, they are not suitable for polymer based documents such as plastic banknotes. This appears to be because polymer documents respond differently to normal wear and tear and the conventional apparatus is unable to simulate this. With the new apparatus, the pushing member not only causes relative rubbing movement between the document and the rubbing pad but also causes the document to flex at a plurality of positions along its length. In practice, the rubbing pad is nearly stationary and the document moves against the pad to give a rubbing action. This makes the apparatus suitable for both paper and polymer documents. Furthermore, providing a pocket in which the document is inserted in use ensures that good rubbing action is obtained between the document and the rubbing pad which causes wear over the creases produced by crumpling. In addition, the use of the pocket and separate attachment portions enables the full document to be tested.

In a preferred apparatus, the apparatus further comprises a resilient pad, wherein the document pocket is tensioned in use across the resilient pad and wherein the pushing member is mounted so as to urge portions of the pocket, document and rubbing pad adjacent the pushing member towards the resilient pad.

Typically, the resilient pad is made of rubber or a rubber-based material while the rubbing pad is conveniently made of a rubberized cotton cloth. A suitable material is known as intaglio fronting cloth as used on conventional impression cylinders.

The rubbing pad may be non-absorbent but is preferably semi-absorbent or fully absorbent. The rubbing pad may contain a water-based liquid such as artificial sweat solution. Other liquids could also be used.

The pocket material is typically a fabric such as nylon. The size of the pocket is chosen to restrict movement of the rubbing pad whilst still allowing slight movement to permit the rubbing effect. The tension applied across the pocket and coefficient of friction of the rubberized surface of the rubbing pad restricts the movement of the rubbing pad while allowing slight movement to permit the rubbing effect. The size of the pocket is chosen to conveniently accommodate the document and allow easy alignment of the document and rubbing pad.

Typically, the apparatus further comprises a clamp for damping one of the attachment portions so as to tension the pocket. The other attachment portion may simply be connected to a fixed anchorage.

In the preferred example, the pushing member is mounted to undergo reciprocating movement along the pocket. In this case, the pushing member may comprise a rotatably mounted toothed wheel which rotates passively as it is reciprocated. Each tooth will cause the pocket and document to flex.

Conveniently, the toothed wheel is mounted on a reciprocally movable trolley which can be moved in any conventional manner. A preferred approach is to connect the trolley to a reversing motor via a push-pull drive chain.

In another example, the pushing member is rotatably mounted and juxtaposed relative to a second roller arranged such that the pocket can be moved about the pushing member and second roller along an S-shaped path.

In this case, the pushing member is preferably also a toothed wheel while the second roller preferably has a smooth surface.

Some examples of testing apparatus according to the invention will now be described with reference to the accompanying drawings, in which:-
Figure 1 is a schematic cross-section through a first example of the apparatus;
Figure 2 is a plan of the wallet assembly shown in Figure 1;
Figure 3 illustrates the pocket on the roller of Figure 1 in more detail; and,
Figure 4 is a schematic view of a second example.

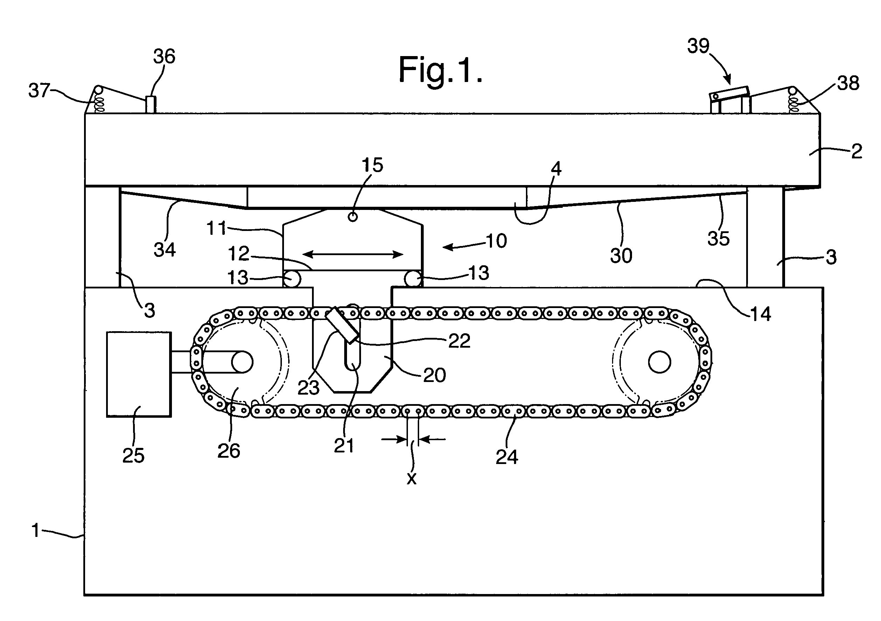

The apparatus shown in Figure 1 comprises a base 1 on which is supported a platform 2 via legs 3. A resilient rubber pad 4 with preferably Shore A hardness 60 and 190mm long x 90mm wide x 13mm thick is secured by adhesive to the underside of the platform 2.

A trolley 10 comprises a pair of opposed side plates 11 (only one shown) secured to a base mount 12. The base mount 12 is mounted on wheels 13 so as to be movable along an upper surface 14 of the base 1.

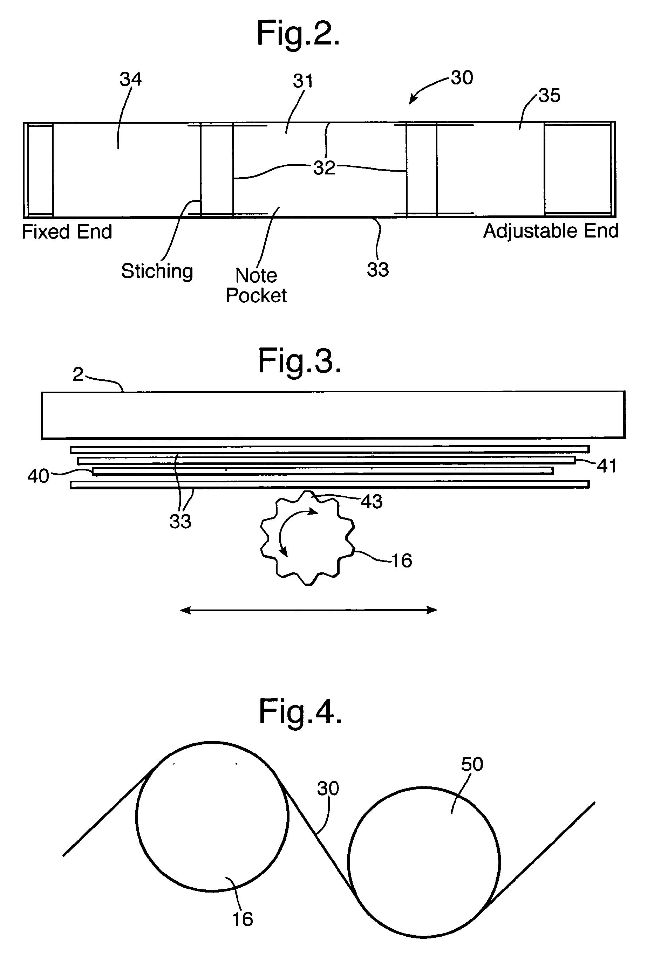

An axle 15 extends between the side plates 11 and a toothed roller 16 (Figure 3) is rotatably mounted on the axle. The toothed roller is made of brass or aluminium and has teeth which protrude above the top of the side plates 11. Typically, the diameter of the toothed roller is ~10mm and there are 10 teeth on the roller. Each tooth preferably has a flattened profile.

A depending plate 20 is connected to the underside of the base mount 12 and extends through a slot (not shown) in the upper surface 14 of the base 1. The plate 20 is provided with a slot 21 within which is located a drive peg 22. The drive peg 22 is attached to a coupling device 23 which is attached to a drive chain 24. The drive chain is driven in an anti-clockwise direction by an electric motor 25, which is coupled to sprocket 26 via two gear wheels (not shown). The trolley is pulled by the drive peg 22 when the coupling device 23 is on the upper side of its path. When the coupling device 23 turns round the sprocket 26, the drive peg 22 moves down the slot 21. The trolley is then pushed by the drive peg 22 as the coupling device 23 moves on the lower side of its path.

A nylon wallet (Figure 2) 30, preferably made from rip-stop nylon spinnaker sail material, includes a pocket 31 having closed sides 32 and open sides 33 into which a note and semi-absorbent rubbing pad can be inserted. The semi-absorbent rubbing pad is a rubberized cotton cloth in which the cotton cloth is coated with rubber on one side which is then pressed into the cotton for adhesion. Typically, the cloth is about 0.5mm thick. Attachment portions 34,35 are provided at each end of the pocket and secured by suitable stitching. Of course, the wallet could be made from one piece of folded material, the pocket 31 simply being defined by stitching.

As can be seen in Figure 1, the attachment portion 34 is attached to an anchorage 36 secured to the platform 2, the attachment portion 34 extending over a spring loaded tensioner 37.

The other attachment portion 35 extends about a second spring loaded tensioner 38 and is manually clamped by a clamp 39 so that the pocket 33 is tensioned against the rubber pad 4.

Figure 3 illustrates the key components of the apparatus in use. Thus, a banknote 40 is inserted into the pocket 31 together with a semi-absorbent rubbing pad 41 impregnated with artificial sweat solution. The toothed pulley 16 is shown in Figure 3 although this is schematic and in practice that part of the pocket 33 adjacent the uppermost tooth 43 will be urged against the rubber pad 2 hence deforming or flexing the banknote 40 and semi-absorbent rubbing pad 41.

In the preferred method, the artificial sweat solution is comprised as follows:
- 5.0g sodium chloride
- 5.0g disodium hydrogen orthophosphate anhydrous
- 3.0ml 0.1N sodium hydroxide
- make up to 1 litre with distilled water
- Final pH should be 9.5 +/- 0.5

An example of the steps involved in carrying out a full durability test is set out below.

The full sweat test consists of five 30 minute test cycles.

For each cycle:
1. Fold note vertically at 1/4, 1/2 and 3/4 distances along its length so that the side to be tested is on the outside, and then unfold.
2. Roll note up, and crumple in a suitable whole-note crumpler.
3. Repeat folding and crumpling.
4. Soak semi-absorbent rubbing pad in artificial sweat solution for 2 minutes.
5. Remove excess artificial sweat solution, and then put the semi-absorbent rubbing pad between 4 paper towels. Use a rubber roller 20 times to further remove sweat from semi-absorbent rubbing pad.
6. Arrange crumpled note 40 and semi-absorbent rubbing pad 41 in pocket 33.
7. Pull wallet taut, and clamp.
8. Run motor 23 for 30 minutes.

As the motor 25 is run, the trolley 10 is slowly moved to and fro. Since the toothed pulley 16 is not driven, it will rotate passively as it urges successive portions of the pocket and banknote to flex thus bringing adjacent teeth to bear on the pocket. In a typical example, the trolley moves at a speed of 10m/minute while the trolley path length is ~21cm. In a 30 minute test cycle, the note will pass across the toothed roller approximately 1500 times.

The movement of the toothed wheel 16 also causes rubbing movement between the semi-absorbent rubbing pad 41 and the banknote 40. However, the longitudinal dimension of the pocket 33 is such that the semi-absorbent rubbing pad 41 can only move a limited distance thus ensuring that it remains wholly in contact with the banknote at all times.

A second example of the apparatus is partly shown schematically in Figure 4. In this case, the trolley 10 (not shown) carries both the toothed wheel 16 and a second, smooth surface wheel 50. The wallet 30 is threaded around the two wheels 16,50 and tensioned as in the first example. The trolley 10 is then moved to and fro as before so that the note and rubbing pad are urged around an S-shaped path.

In a modified form of this example, the wheel 16 is smooth surfaced.

## Claims

1. Document durability testing apparatus comprising:
a document pocket, in which a document and rubbing pad are inserted in use, the document pocket being made of a flexible material and being coupled with attachment portions at each end so that the pocket can be tensioned; and
a rubbing system including a pushing member which engages and moves in use relative to the document pocket, and which is mounted so as to urge portions of the pocket, document and rubbing pad adjacent the pushing member away from the pushing member thereby also causing relative rubbing movement between the document and the rubbing pad in the pocket.

2. Apparatus according to claim 1, further comprising a resilient pad, wherein the document pocket is tensioned in use across the resilient pad and wherein the pushing member is mounted so as to urge portions of the pocket, document and rubbing pad adjacent the pushing member towards the resilient pad.

3. Apparatus according to claim 2, wherein the resilient pad comprises rubber or a rubber-based material.

4. Apparatus according to claim 2 or claim 3, further comprising a clamp for clamping one of the attachment portions so as to tension the pocket.

5. Apparatus according to any of claims 2 to 4, wherein the pushing member is mounted to undergo a reciprocal movement along the pocket.

6. Apparatus according to claim 5, wherein the pushing member comprises a rotatably mounted toothed wheel.

7. Apparatus according to claim 6, wherein the toothed wheel is mounted on a reciprocally movable trolley.

8. Apparatus according to claim 7, wherein the trolley is connected to a reversible motor via a push-pull drive chain.

9. Apparatus according to claim 1, wherein the pushing member is rotatably mounted and juxtaposed relative to a second roller arranged such that the pocket can be moved about the pushing member and second roller along an S-shaped path.

10. Apparatus according to claim 9, wherein the pushing member is toothed.

11. Apparatus according to claim 9 or claim 10, wherein the second roller has a smooth outer surface.

12. Apparatus according to any of the preceding claims, wherein the rubbing pad is an absorbent or semi-absorbent pad such as a rubberized cotton cloth,

13. Apparatus according to any of the preceding claims, wherein the rubbing pad contains a water-based liquid such as artificial sweat solution.

14. Apparatus according to any of the preceding claims, wherein the pocket material is a fabric such as nylon.

15. Apparatus according to any of the preceding claims, wherein the pocket has a size in the rubbing direction slightly greater than the size of the rubbing pad.
